(19) Europäisches Patentamt
European Patent Office
Office européen des brevets

(11) Veröffentlichungsnummer: **0 280 156 B1**

(12) **EUROPÄISCHE PATENTSCHRIFT**

(45) Veröffentlichungstag der Patentschrift: **11.11.92**

(21) Anmeldenummer: **88102229.7**

(22) Anmeldetag: **16.02.88**

(51) Int. Cl.[5]: **C07C 231/00**, C07C 233/02, C07C 233/16, C07C 235/74, C07C 303/40, C07C 255/49, C07D 213/75, C07D 215/38, C07D 217/22

# (54) Verfahren zur Herstellung von Acetoacetylarylamiden bzw. -heteroarylamiden desaktivierter Aromaten.

(30) Priorität: **25.02.87 DE 3706009**
**23.09.87 DE 3731912**

(43) Veröffentlichungstag der Anmeldung:
**31.08.88 Patentblatt 88/35**

(45) Bekanntmachung des Hinweises auf die Patenterteilung:
**11.11.92 Patentblatt 92/46**

(84) Benannte Vertragsstaaten:
**AT BE CH DE ES FR GB IT LI NL SE**

(56) Entgegenhaltungen:
**EP-A- 0 004 611**
**EP-A- 0 053 713**
**DE-A- 2 519 036**
**GB-A- 715 896**

**CHEMICAL PHARMACEUTICAL BULLETIN, Band 29, Nr. 4, 1981, Seiten 1049-1055; H. YAMANAKA et al, "Studies on Ouinoline and Isoquinoline Derivatives. VI. Addition Reaction of Diketene with Isoquinolines in the Presence of Carboxylic Acids"**

**THE JOURNAL OF GENERAL CHEMISTRY OF THE USSR, Band 21, 1981, Seiten 1995-2000; V. V. PEREKALIN et al, "Reactions of ketene dimer; Reaction of ketene dimer with some weakly basic aromatic amines"**

(73) Patentinhaber: **HOECHST AKTIENGESELLSCHAFT**
**Postfach 80 03 20**
**W-6230 Frankfurt am Main 80(DE)**

(72) Erfinder: **Papenfuhs, Theodor, Dr.**
**Heinrich-Bleicher-Strasse 40**
**W-6000 Frankfurt am Main 50(DE)**
Erfinder: **Daub, Wolfgang, Dr.**
**Münsterer Strasse 37a**
**W-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Gegenstand der Erfindung ist ein gegenüber den bekannten Verfahren wesentlich verbessertes Verfahren zur Herstellung von Acetoacetylarylamiden bzw. -heteroarylamiden desaktivierter Aromaten bzw. Heteroaromaten.

Bekanntlich sind Acetoacetylarylamide schiebend substituierter (aktivierter) Aromaten relativ problemlos in guten Ausbeuten und hohen Reinheiten aus Diketen und den entsprechenden Anilinderivaten zugänglich. Hierbei wird oft in besonders wirtschaftlicher Weise direkt in Wasser oder in wäßriger Essigsäure mit einem geringen Überschuß an Diketen gearbeitet [EP 0053713 B1].

In HOUBEN-WEYL, Band XI/2, Seite 19 wird angegeben, daß schwach basische aromatische Amine, d. h. ziehend substituierte aromatische Amine, sich nur schwierig mit Diketen umsetzen lassen. An der genannten Stelle heißt es unter Bezugnahme auf drei Literaturstellen ferner, daß aromatische Amine der genannten Art bei Gegenwart katalytischer Mengen eines tertiären aliphatischen Amins oder von Pyridin angeblich leicht mit Diketen reagieren bzw. daß die Acetoacetylierung derartiger Amine in 90 - 100 %iger Essigsäure in guten Ausbeuten gelinge. Die genaue Durchsicht dieser drei Literaturstellen zeigt jedoch, daß die beschriebenen Verfahren schwerwiegende Mängel aufweisen. So liefert das in Zh. Obsch. Khim. 21, 1995 (1951) von W.W. Perekalin und O.M. Lerner beschriebene Verfahren die acetoacetylierten aromatischen Amine nur in mäßigen Ausbeuten. Hinzu kommt, daß als Lösungsmittel siedendes Aceton eingesetzt wird, das aufgrund seines extrem niedrigen Flammpunktes einen hohen technischen Aufwand erfordert. In der zweiten dort angegebenen Literaturstelle (Britische Patentschrift 715 896) wird ein ähnliches Verfahren beschrieben, wobei unter Verwendung des gesundheitsschädlichen Benzols als Lösungsmittel in der Regel ebenfalls unbefriedigende Ausbeuten erzielt werden. Außerdem läßt ein Vergleich der dort angegebenen Schmelzpunkte mit anderen Literaturdaten den Schluß auf eine geringe Produktqualität zu. Gemäß dem in der dritten dort zitierten Literaturstelle (Deutsche Patentschrift 926 130) beschriebenen Verfahren werden vor allem Aminoanthrachinone mit Diketen in Essigsäure umgesetzt. Dieses Verfahren ist jedoch mit dem schwerwiegenden Nachteil behaftet, daß in der Regel sehr lange Reaktionszeiten und sehr große Diketenüberschüsse angewandt werden müssen, was üblicherweise zur Bildung von Nebenprodukten führt (T. Kato, Acc. Chem. Res. 7, 265 (1974)) und die teilweise schlechte Produktqualität erklärt.

Aufgrund der genannten Mängel sind die aus den vorstehend aufgeführten Literaturstellen bekannten Verfahren für eine technische Nutzung nicht geeignet.

In TETRAHEDRON 22, 2003 (1966) wird von Gunar et al. ein Verfahren beschrieben, das die Herstellung einiger Acetoacetylarylamide in guten Ausbeuten und hohen Reinheiten erlaubt. Der Nachteil dieses Verfahrens liegt jedoch in der Verwendung hochgiftiger Quecksilbersalze als Katalysatoren. Außerdem wird in der zwischenzeitlich veröffentlichten US-Patentschrift 3708580 gezeigt, daß bei stärker desaktivierten Anilinen die Ausbeuten stark zurückgehen.

Bekannt war auch, daß die katalytische Wirkung tertiärer Amine in aprotischen Lösungsmitteln, wie Aceton, Petrolether, Benzol oder Toluol, sehr wahrscheinlich auf der intermediären Bildung eines Komplexes der nachstehenden Formel (3) aus Diketen und Amin beruht (HOUBEN-WEYL VII/4, Seite 228):

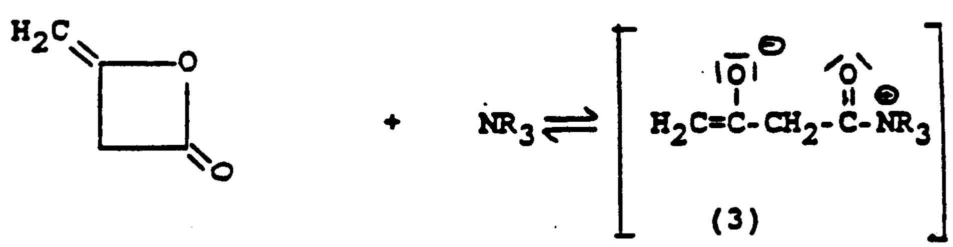

(3)

In der Literatur finden sich jedoch keinerlei Hinweise auf Versuche, diesen Effekt auch auf protische Systeme zu übertragen. Dies ist verständlich, da einerseits Diketen unter solchen Bedingungen Nebenreaktionen mit den zwangsläufig vorhandenen anionischen Gegenionen eingehen kann und andererseits der Katalysator protoniert oder zumindest solvatisiert und dadurch in seiner Wirkung eingeschränkt würde.

Es wurde nun überraschenderweise gefunden, daß man Acetoacetylarylamide bzw. -heteroarylamide desaktivierter Aromaten bzw. Heteroaromaten der allgemeinen Formel (1)

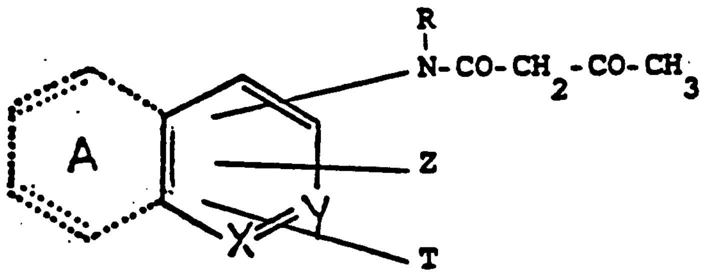

(1)

in welcher X und Y je CH bedeuten oder eines der beiden Ringglieder X und Y ein Stickstoffatom darstellt, A einen anellierten Benzolring bedeutet, der ebenfalls durch T, Z und

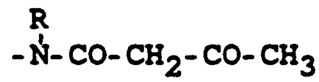

substituiert sein kann, R ein Wasserstoffatom, eine lineare oder verzweigte Alkylgruppe von 1 bis 6 Kohlenstoffatomen, eine Cycloalkylgruppe von 5 oder 6 Kohlenstoffatomen, die durch 1 bis 3 Alkylgruppen von 1 bis 4 Kohlenstoffatomen substituiert sein kann, eine β-Hydroxyethylgruppe, Benzylgruppe oder eine Phenylgruppe, die durch Fluor-, Chlor-, Brom- und/oder Jodatome und/oder eine Nitrogruppe und/oder lineare oder verzweigte Alkylgruppen und/oder Alkoxygruppen von jeweils 1 bis 4 Kohlenstoffatomen substituiert sein kann, bedeutet, Z ein Fluor-, Chlor-, Brom- oder Jodatom oder eine Gruppe aus der Reihe m-OR′, -OCOR′, $-SO_3R'$,$-SO_2NR'R''$, $-SO_2R'$, -SR′, $-NO_2$, -NH-CO-R′, $-NH-CO-CH_2-CO-CH_3$, $-PO(OR')_2$, -PO-$(NR'R'')_2$, -COOR′, -CN, -CONR′R″, -COR′, Halogenalkoxy mit 1 bis 4 Kohlenstoffatomen, Halogenalkyl mit 1 bis 8 Kohlenstoffatomen, vorzugsweise $-CF_3$, wobei Halogen jeweils Fluor, Chlor oder Brom bedeutet, oder eine Phenylgruppe, die durch Fluor-, Chlor-, Brom- und/oder Jodatome und/oder Nitro-, Cyano-, -CO-R′, -COOR′-Gruppen substituiert sein kann, bedeutet, oder auch ein Wasserstoffatom darstellen kann, wenn X oder Y ein Stickstoffatom oder R in Formel (1) eine gegebenenfalls substituiert Phenylgruppe bedeutet, wobei R′ und R″ Wasserstoffatome, lineare oder verzweigte Alkylgruppen von 1 bis 6 Kohlenstoffatomen, Cycloalkylgruppen von 5 oder 6 Kohlenstoffatomen, die durch 1 bis 3 Alkylgruppen von 1 bis 4 Kohlenstoffatomen substituiert sein können, β-Hydryxyethylgruppen, Benzylgruppen oder Phenylgruppen, die am aromatischen Kern durch Fluor-, Chlor-, Brom- oder Jodatome und/oder Nitrogruppen und/oder lineare oder verzweigte Alkylgruppen und/oder Alkoxygruppen von jeweils 1 bis 4 Kohlenstoffatomen substituiert sein können, darstellen, T ein Wasserstoffatom, eine oder mehrere lineare oder verzweigte Alkylgruppen oder Alkoxygruppen von 1 bis 6 Kohlenstoffatomen oder eine Cycloalkylgruppe von 5 bis 6 Kohlenstoffatomen, die durch 1 bis 3 Alkylgruppen von 1 bis 4 Kohlenstoffatomen substituiert sein kann, eine Aminogruppe, die durch zwei Alkylgruppen von 1 bis 4 Kohlenstoffatomen substituiert ist, eine β-Hydroxyethylgruppe oder einen bis zwei, im Falle von Fluor, Chlor und/oder Brom auch mehr als zwei der für Z genannten Substituenten bedeutet, in guten Ausbeuten und in hoher Reinheit vorteilhaft herstellen kann, indem man 1 Mol eines Amins der allgemeinen Formel (2)

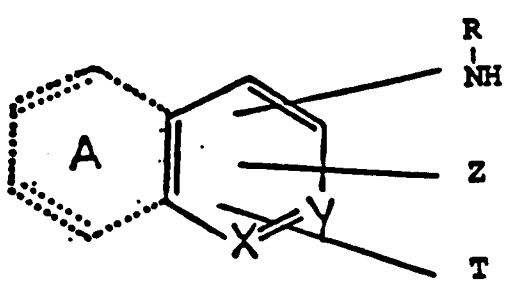

(2)

in welcher A, R, T, X, Y und Z die vorstehend genannten Bedeutungen haben, mit 1 bis 1,2 Mol, vorzugsweise 1,05 bis 1,1 Mol, Diketen bei Temperaturen von 20 bis 100 ° C, vorzugsweise 50 bis 80 ° C, in Eisessig in Gegenwart von 1 bis 20 Molprozent, vorzugsweise 5 bis 10 Molprozent, eines basischen Katalysators, bezogen auf das eingesetzte Amin der genannten allgemeinen Formel (2), umsetzt.

Als geeignete basische Katalysatoren kommen sowohl tertiäre Amine als auch Anionen in Frage.

Geeignete tertiäre Amine sind beispielsweise Trialkylamine mit geradkettigen oder verzweigten Alkylgruppen von 1 bis 9 Kohlenstoffatomen, Cycloalkylgruppen von 5 oder 6 Kohlenstoffatomen, die durch 1 bis 3 Alkylgruppen von 1 bis 4 Kohlenstoffatomen substituiert sein können, Benzylgruppen oder $\beta$-Hydroxyethylgruppen, wie beispielsweise Triethylamin, Tributylamin, Trioctylamin, Diethylcyclohexylamin oder Dimethyl-$\beta$-hydroxyethylamin, ferner Dialkylarylamine mit Alkylgruppen von 1 bis 6 Kohlenstoffatomen, wobei die Alkylgruppen geradlinig oder verzweigt sein können und auch Cycloalkylgruppen von 5 oder 6 Kohlenstoffatomen, die durch 1 bis 3 Alkylgruppen von 1 bis 4 Kohlenstoffatomen substituiert sein können, darstellen können, und wobei die Arylgruppe ein Phenyl-, Alkyl$C_1$-$C_6$phenyl-, Alkoxy$C_1$-$C_4$phenyl- oder Di-(alkyl$C_1$-$C_4$)aminophenyl-Rest sein kann, wie beispielsweise N,N-Dimethylanilin, ferner ein N-Alkyl$C_1$-$C_6$pyrrolidin, wobei der Alkylrest auch ein Cycloalkylrest von 5 bis 6 Kohlenstoffatomen, der durch 1 bis 3 Alkylgruppen von 1 bis 4 Kohlenstoffatomen substituiert sein kann, wie ein Cyclopentyl- oder Cyclohexylrest, sein kann, ein N-Alkyl$C_1$-$C_6$piperidin, wobei der Alkylrest auch ein Cycloalkylrest von 5 bis 6 Kohlenstoffatomen sein kann, der durch 1 bis 3 Alkylgruppen von 1 bis 4 Kohlenstoffatomen substituiert sein kann, ferner Pyridin, Monoalkyl$C_1$-$C_4$pyridin, wie beispielsweise 2-, 3- oder 4-Methylpyridin oder 2-, 3- oder 4-Ethylpyridin, 2,3-, 2,4-, 2,5-, 2,6-, 3,4- oder 3,5-Dialkyl$C_1$-$C_4$pyridin, wie beispielsweise 2,3-, 2,4-, 2,5, 2,6-, 3,4- oder 3,5-Dimethylpyridin, 2,4,6-Trialkyl$C_1$-$C_4$-pyridin, wie beispielsweise 2,4,6-Trimethylpyridin, 4-Dimethylaminopyridin, 4-Diethylaminopyridin, Chinolin, Isochinolin, N-Alkyl$C_1$-$C_4$morpholin, wie beispielsweise N-Methylmorpholin oder N-Ethylmorpholin, Triethylendiamin, Tris(3,6-dioxaheptyl)amin oder N,N,N',-N'Tetraalkyl$C_1$-$C_4$alkylen$C_1$-$C_4$diamin, wie beispielsweise N,N,N',N'Tetramethyl- oder Tetraethylethylendiamin. Die genannten tertiären Amine können auch in Mischung eingesetzt werden.

Geeignete anionische Katalysatoren sind beispielsweise Anionen aus der Reihe Fluorid, Acetat, Trifluormethansulfonat, Trifluoracetat, Benzoat, o-, m- bzw. p-Alkyl$C_1$-$C_4$benzoat, o-, m- bzw. p-Alkoxy-$C_1$-$C_4$benzoat und o- m- bzw. p-Di(alkyl$C_1$-$C_4$amino)-benzoat. Die vorstehend genannten Anionen werden zweckmäßigerweise in Form der Alkalimetallsalze, vorzugsweise der Natrium-und Kaliumsalze, angewandt. Die genannten Anionen können auch in Mischung zur Anwendung gelangen.

Es ist auch möglich, ein tertiäres Amin (Katalysator) zusammen mit einem anionischen Katalysator anzuwenden.

An geeigneten Reaktionskomponenten (Aminen) der genannten allgemeinen Formel (2) seien beispielsweise folgende genannt:

2-, 3- und 4-Fluoranilin, 2-, 3- und 4-Chloranilin, 2-, 3- und 4-Bromanilin, 2-, 3- und 4-Jodanilin, 2,3-Difluoranilin, 2,4-Difluoranilin, 2,5-Difluoranilin, 2,6-Difluoranilin, 3,4-Difluoranilin, 3,5-Difluoranilin, 2,3-Dichloranilin, 2,4-Dichloranilin, 2,5-Dichloranilin, 2,6-Dichloranilin, 3,4-Dichloranilin, 3,5-Dichloranilin, 2,4-Dibromanilin, 2,5-Dibromanilin, 2,6-Dibromanilin, 3-Chlor-4-fluoranilin, 4-Brom-2-chloranilin, 2-Brom-4,6-difluoranilin, 2,3,4-Trichloranilin, 2,4,5-Trichloranilin, 2,4,6-Trichloranilin, 3,4,5-Trichloranilin, 2,4,6-Trifluoranilin, 2,4,6-Tribromanilin, 2,3,4,5-Tetrachloranilin, 2,3,5,6-Tetrafluoranilin, Pentafluoranilin, 2-, 3- und 4-Nitroanilin, 2,4-Dinitroanilin, 3,5-Dinitroanilin, 2-Fluor-5-nitroanilin, 4-Fluor-2-nitroanilin, 4-Fluor-3-nitroanilin, 4,5-Difluor-2-nitroanilin, 4-Chlor-2-nitroanilin, 2-Chlor-4-nitroanilin, 2-Chlor-5-nitroanilin, 3-Chlor-4-nitroanilin, 3-Chlor-5-nitroanilin, 2-Chlor-6-nitroanilin, 4-Chlor-3-nitroanilin, 5-Chlor-2-nitroanilin, 4-Brom-2-nitroanilin, 2-Brom-5-nitroanilin, 2-Brom-4-nitroanilin, 5-Chlor-2,4-dinitroanilin, 6-Chlor-2,4-dinitroanilin, 2,4-Dichlor-6-nitroanilin, 2,6-Dichlor-4-nitroanilin, 4,5-Dichlornitroanilin, 4,6-Dichlor-2-nitroanilin, 2,5-Dichlor-4-nitroanilin, 2-Chlor-4-nitro-6-bromanilin, 2,4-Dibrom-6-nitroanilin, 2,6-Dibrom-4-nitroanilin, 4,6-Dibrom-2-nitroanilin, 2,6-Dijod-4-nitroanilin, 3-Methoxyanilin, 4-Methoxy-2-nitroanilin, 2-Methoxy-5-nitroanilin, 4-Methoxy-3-nitroanilin, 2-Methoxy-4-nitroanilin, 3-Methoxy-4-nitroanilin, 4-Ethoxy-2-nitroanilin, 4-Butoxy-2-nitroanilin, 2,5-Dimethoxy-4-nitroanilin, 3-Chlor-4-ethoxy-6-nitroanilin, 2-Chlor-5-methoxy-4nitroanilin, 2,4-Dinitro-5-methoxyanilin, 4-Chlor-5-methoxy-2-nitroanilin, 2,4-Dichlor-5-methoxyanilin, 3-Methoxy-5-nitroanilin, 5-Chlor-2-(2-chlorphenoxy)-anilin, 5-Chlor-2-(4-chlorphenoxy)anilin, 2,4-Dichlor-6-phenoxyanilin, 5-(2',3'-Dichlorphenoxy)-4-chlor-2-nitranilin, 4,4'-Dichlor-2-aminodiphenylether, 4-Hydroxy-3-nitroanilin, 5-Hydroxy-2-nitroanilin, 2-Hydroxy-4-nitroanilin, 5-Chlor-2-hydroxy-4-nitroanilin, 5-Chlor-2-hydroxy-3-nitroanilin, 3-Chlor-2-hydroxy-5-nitroanilin,4-Hydroxy-2-nitroanilin, 4-Methyl-2-nitroanilin, 2-Methyl-6-nitroanilin, 4-Methyl-3-nitroanilin, 2-Methyl-5-nitroanilin, 2-Methyl-4-nitroanilin, 2,4-Dimethyl-6-nitroanilin, 2,3-Dimethyl-4-nitroanilin, 2,5-Dimethyl-4-nitroanilin, 2-Chlor-5-($\beta$-hydroxyethyl)-4-nitroanilin, 5-Chlor-2-methyl-4-nitroanilin, 6-Chlor-2-methyl-4-nitroanilin, 2,6-Dichlor-3-methylanilin, 3,5-Dichlor-4-hydroxyanilin, 4-Chlor-2-methyl-6-nitroanilin, 2-Brom-6-methyl-4-nitroanilin, 3,5-Dinitro-2,3,6-trimethylanilin, 2-Methoxy-4-nitro-5-methylanilin, 5-Methoxy-4-nitro-2-methylanilin, 6-Methoxy-3-methyl-2,4-dinitroanilin, 4-Nitro-2-aminodimethylanilin, 2-, 3- und 4-(Trifluormethyl)anilin, 4-Fluor-2-(trifluormethyl)anilin, 2-Fluor-5-(trifluormethyl)anilin, 4-Fluor-3-(trifluormethyl)anilin, 2-Brom-5-(trifluormethyl)-anilin, 3,5-Bis(trifluormethyl)-anilin, 2-Nitro-4-(trifluormethyl)anilin, 4-Nitro-2-(trifluormethyl)anilin, 4-Nitro-3-(trifluormethyl)anilin, 2-Chlor-4-nitro-6-(trifluormethyl)anilin, 2-Chlor-3-nitro-5-(trifluormethyl)anilin, 4-Chlor-2-(trifluormethyl)anilin, 2,4-Dichlor-6-(trifluormethyl)anilin, 2-Chlor-5-(trifluormethyl)anilin, 2-, 3- und 4-Aminobenzoesäure, 2-, 3- und 4-Aminobenzoesäuremethylester, -ethylester, -butylester, -benzylester, 4-Aminophthalsäure, 4-Aminophthalsäuredimethylester, 5-Aminoisophthalsäuredimethylester, 4-Amino-3,5-dijodbenzoesäuremethylester, 4-Amino-3-nitrobenzoesäureethylester, 4-Amino-3-nitro-5-brombenzoesäureethylester, 2-Amino-5-nitrobenzoesäure, 4-Amino-3-nitrobenzoesäure, 2-Amino-4-nitrobenzoesäuremethylester, 5-Amino-2-nitrobenzoesäure, 2-, 3- und 4-Aminobenzonitril, 4-Amino-2-chlorbenzonitril, 2-Amino-5-chlorbenzonitril, 2-Hydroxybenzoesäure-(4-aminophenyl)ester, 2-Amino-4-(trifluormethyl)benzonitril, 2-Amino-3,5-dichlorbenzoesäuremethylester, 4-Amino-3-nitrobenzonitril, 2-Amino-5-nitrobenzonitril, 3-Aminobenzoesäuredimethylamide, 3-Aminobenzoesäure-di-n-butylamid, 3-Aminobenzoesäureanilid, 3-Amino-4-methoxybenzoesäureanilid, 3-Aminobenzoesäure-2′-chloranilid, 3-und 4-Aminoacetophenon, 3- und 4-Aminobenzophenon, 2-, 3-und 4-Aminoacetylacetamid, 2-, 3- und 4-Aminoacetanilid, 3-Aminopropionylanilid, 3-Aminoformanilid, 4-Amino-1-benzoylaminobenzol, 4-Chlor-2-aminoacetanilid, 4-Amino-3-nitroacetanilid, 4-Amino-3-nitroacetylacetanilid, Anilin-2-sulfonsäuredimethylamid, Anilin-3-sulfonsäurephenylester, Anilin-3-sulfonsäure-bis-(β-hydroxyethylamid), 3-(Tetrafluorethoxy)anilin, 3-(Perfluoroctyl)anilin, 3-(Perfluorhexyl)anilin, 2-Nitroanilin-4-sulfonsäureamid, 2-Chloranilin-5-sulfonsäurecyclohexylamid, 3-Amino-4-methoxybenzolsulfonsäurediethylamid, 2,6-Dichlor-4-(methylsulfonyl)anilin, 2-Methylsulfonyl-4-nitroanilin, 2-Aminodiphenylsulfon, 3-(Methylthio)anilin, 3-Aminophenylphosphonsäurediethylester, 3-Aminophenylphosphonsäure-bis-(diethylamid), 1-Amino-4-chlornaphthalin,1-Amino-4-nitronaphthalin,1-Amino-2-nitronaphthalin, 1-Amino-5-nitronaphthalin, Diphenylamin, 3-Methoxydiphenylamin, 4-Ethoxydiphenylamin, 2-Nitrodiphenylamin, 4-Nitrodiphenylamin, 4′-Methoxy-4-nitro-3-(trifluormethyl)diphenylamin, 3-Hydroxydiphenylamin, N-Methyl-4-nitroanilin, N-Methyl-3-nitroanilin, N-n-Butyl-4-nitroanilin, N,2-Dimethyl-5-nitroanilin, N-Methyl-2,4-dinitroanilin, N-Benzyl-4-nitroanilin, 2-, 3- und 4-Aminopyridin, 3-Amino-2-nitropyridin, 2-Amino-3,5-dibrompyridin, 2-Amino-3,5-dichlorpyridin, 3-Amino-2-chlorpyridin, 5-Amino-2-chlorpyridin, 3-Amino-2,6-dimethoxypyridin, 2-Amino-4,6-dimethylpyridin, 5-Amino-2-methoxypyridin, 4-Amino-2,3,5,6-tetrafluorpyridin, 3-Aminochinolin, 5-Amino-6-nitrochinolin und 5-Aminoisochinolin.

Hinsichtlich Einzelheiten des erfindungsgemäßen Verfahrens sei noch folgendes ausgeführt:
Die Anwendung der genannten Katalysatoren bewirkt eine starke Beschleunigung der Umsetzung von Aminen der genannten allgemeinen Formel (2) mit Diketen, so daß in der Regel eine Reaktionszeit von $\frac{1}{2}$ bis 3 Stunden ausreicht, um gute bis sehr gute Ausbeuten (meist 85 bis 95 % der Theorie) und hervorragende Produktqualitäten zu erzielen.

Geringe Mengen Wasser im Reaktionsgemisch stören nicht; größere Mengen Wasser können jedoch zu einem erhöhten Diketen-Verbrauch und zu geringerer Produktqualität führen.

Ein besonderer Vorteil des erfindungsgemäßen Verfahrens liegt darin, daß daz Zielprodukt in der Regel nach Abkühlen auf etwa 20 bis 40 °C, gegebenenfalls nach vorangegangener Klärung, einfach durch Zugabe von Wasser bzw. Eiswasser ausgefällt werden kann, oder, noch besser, bei geeigneter Wahl der Eduktkonzentration beim Abkühlen ohne Verdünnung auskristallisiert und die gesättigte Mutterlauge nach Abtrennung ganz oder teilweise für das erfindungsgemäße Verfahren wieder eingesetzt werden kann.

Acetoacetylarylamide bzw. Acetoacetylheteroarylamide sind wertvolle Zwischenprodukte, die häufig als Kupplungskomponenten bei der Herstellung von Azofarbstoffen, insbesondere von Azopigmenten, in vielen Fällen aber auch als Vorstufen für Pharmaka und Pflanzenschutzmittel dienen.

Die nachstehenden Beispiele dienen zur Erläuterung des erfindungsgemäßen Verfahrens, ohne es darauf zu beschränken.

**Beispiel 1**

N-(4-Nitrophenyl)acetylacetamid (1a):

69,1 g (0,50 Mol) p-Nitranilin und 10 g Tri-n-butylamin werden in 500 g Eisessig bei ca. 70 °C vorgelegt. Innerhalb 30 Minuten werden 46,5 g (0,55 Mol) Diketen zugetropft. Die Temperatur steigt dabei ohne weiteres Heizen auf etwa 80 °C an.
Nach insgesamt 1 Stunde bei 70 bis 80 °C ist die Umsetzung beendet. Das Produkt wird nach Abkühlen durch Zugabe von etwa 1 Liter Eiswasser ausgefällt.

Man erhält 98,0 g N-(4-Nitrophenyl)acetylacetamid (1a) vom Schmelzpunkt 119 bis 122 °C in einer Ausbeute von etwa 88,2 % der Theorie mit einem Reingehalt von 96 % (HPLC). ("HPLC" bedeutet "high performance liquid chromatography".)

**Beispiele 2 - 29**

Die in der nachfolgenden Tabelle aufgelisteten Umsetzungen wurden gemäß Beispiel 1 durchgeführt. Variierte Parameter, Ausbeute, Reinheit (nach HPLC) und Schmelzpunkt sind angegeben.

Tabelle 1: Umsetzung desaktivierter aromatischer Aminoverbindungen mit Diketen in Eisessig unter Tri-n-butylamin-Katalyse
(Die Buchstaben T, A, R, X und Z beziehen sich auf die allgemeinen Formeln (1) bzw. (2))

| Beispiel | Amin (nach Formel(2)) | Produkt[1] (nach Formel (1)) | Diketen [Mol] | Temperatur [°C] | Zeit [h] | Ausbeute [% d.Th.] | Reinheit [HPLC/Fl.-%] | Schmp. [°C] |
|---|---|---|---|---|---|---|---|---|
| 2 | o-Nitranilin | 1b | 0,55 | 67 - 71 | 2,7 | 85,2 | 99,1 | 65 |
| 3 | m-Nitranilin | 1c | 0,56 | 69 - 74 | 1,5 | 89,5 | 99,1 | 119 |
| 4 | 4-Chlor-2-nitranilin | 1d | 0,56 | 70 | 1,5 | 94,4 | 98,1 | 83 |
| 5 | 2-Trifluormethylanilin | 1e | 0,57 | 70 - 82 | 1,7 | 95,2 | 96,1 | 48 |
| 6 | 2-Nitro-p-toluidin | 1f | 0,56 | 64 - 73 | 2 | 86,9 | 99,5 | 105 |
| 7 | Antranilsäureethylester | 1g | 0,55 | 48 - 56 | 2 | 96,8 | 95,2 | 61-62 |
| 8 | 4-Nitro-o-anisidin | 1h | 0,55 | 68 - 73 | 2,25 | 82,9 | 98,6 | 110 |
| 9 | 2,4,5-Trichloranilin | 1k | 0,55 | 69 - 71 | 1,7 | 95,5 | 94,6 | 104 |
| 10 | 2-Aminobenzonitril | 1m | 0,53 | 67 - 73 | 2 | 86,3 | 99,3 | 116 |
| 11 | 2-Nitro-p-anisidin | 1n | 0,55 | 71 - 73 | 1,7 | 80,2 | 97,5 | 70 |
| 12 | 2,4-Dinitroanilin | 1p | 0,60 | 70 - 74 | 3 | 60,5 | 96,9 | 109 |

**Tabelle 1 (Fortsetzung):**

| Beispiel | Amin (nach Formel (2)) | Produkt[1] (nach Formel (1)) | Diketen [Mol] | Temperatur [°C] | Zeit [h] | Ausbeute [% d.Th.] | Reinheit [HPLC/Fl.-%] | Schmp. [°C] |
|---|---|---|---|---|---|---|---|---|
| 13 | 1,4-Diaminobenzol [2] | 1q | 1,10 | 69 - 78 | 2 | 81,8 | 99,2 | 177-178 |
| 14 | Diphenylamin | 1r | 0,55 | 70 - 73 | 2,5 | 90,4 | 99,1 | 84-85 |
| 15 | 3-Aminopyridin [3] | 1s | 0,55 | 69 - 72 | 2,5 | 92,9 | 98,1 | 137 |
| 16 | 1-Amino-4-nitro-naphthalin | 1t | 0,55 | 67 - 70 | 2,25 | 75,2 | 99.0 | 150 |
| 17 | 2-Diethylamino-5-nitranilin | 1u | 0,55 | 60 | 2,5 | 81,8 | 97,8 | 143-147 |
| 18 | 4-Amino-3-nitrobenzoesäureethylester | 1v | 0,55 | 68 - 73 | 2,7 | 77,7 | 96,8 | 74-76 |
| 19 | 3,3'-Dichlorbenzidin [4] | 1w | 1,20 | 60 - 67 | 1 | 92,6 | 97,9 | 220 |
| 20 | 3-Nitro-4-aminobenzotrifluorid | 1x | 0,56 | 70 | 2,5 | 92,4 | 99,6 | 109-111 |
| 21 | 3-Amino-4-methoxy-N-phenylbenzamid | 1y | 0,55 | 70 - 74 | 3 | 93,9 | 100 | 202 |
| 22 | (5-Amino-2-chlorphenyl)-(β-hydroxyethyl)sulfon | 1z | 0,55 | 70 | 5 | 83,5 | 99,4 | 84-89 |

**Tabelle 1 (Fortsetzung):**

| Beispiel | Amin (nach Formel (2)) | Produkt[1)] (nach Formel (1)) | Diketen [Mol] | Temperatur [°C] | Zeit [h] | Ausbeute [% d.Th.] | Reinheit [HPLC/Fl.-%] | Schmp. [°C] |
|---|---|---|---|---|---|---|---|---|
| 23 | 4-Chlor-5-(2',3'-dichlorphenoxy)-2-nitranilin | 1aa | 0,55 | 70 | 2,5 | 87,3 | 96,6 | 150 |
| 24 | 2-Cyano-4-nitranilin | 1ab | 0,60 | 70 | 3 | 70,6 | 100 | 169-172 |
| 25 | 2-Brom-4-nitranilin | 1ac | 0,56 | 70 | 2,5 | 74,2 | 98,0 | 98-100 |
| 26 | 4-Nitro-2-trifluormethylanilin | 1ad | 0.60 | 70 | 3.5 | 76.9 | 99.1 | 68-70 |
| 27 | 3-Amino-4-methoxybenzolsulfosäurediethylamid | 1ae | 0.56 | 70 - 75 | 2.5 | 80.2 | 98.7 | 79-80 |
| 28 | 3-(Tetrafluorethoxy)anilin | 1af | 0.56 | 70 - 75 | 2.25 | 78.7 | 100 | 90-91 |
| 29 | 2,5-Dichlor-4-nitroanilin | 1ag | 0.56 | 70 | 3.5 | quantitativ | 96.0 | 95-97 |

**Anmerkungen zu Tabelle 1**

1) Die Identität aller Produkte wurde mittels Elementaranalyse und $^1$H-NMR überprüft.

2) 4-(Acetoacetylamino)anilin wurde nicht zwischenisoliert.

3) Das Produkt ist wasserlöslich. In Abweichung von Beispiel 1 muß daher das Lösungsmittel teilweise abdestilliert werden, worauf das Produkt auskristallisiert und abgesaugt werden kann.

4) 4-(4-Acetoacetylamino-3-chlorphenyl)-2-chloranilin wurde nicht zwischenisoliert.

Die in Tabelle 1 aufgeführten Buchstaben haben für die Substituenten T, R, X, A und Z in der allgemeinen Formel (1) folgende Bedeutungen:

| | | | |
|---|---|---|---|
| a: | Z = 4-$NO_2$ | T = H | |
| b: | Z = 2-$NO_2$ | T = H | |
| c: | Z = 3-$NO_2$ | T = H | |
| d: | Z = 2-$NO_2$ | T = 4-Cl | |
| e: | Z = 2-$CF_3$ | T = H | |
| f: | Z = 2-$NO_2$ | T = 4-$CH_3$ | |
| g: | Z = 2-$COOC_2H_5$ | T = T | |
| h: | Z = 4-$NO_2$ | T = 2-$OCH_3$ | |
| k: | Z = 5-Cl | T = 2,4-$Cl_2$ | |
| m: | Z = 2-CN | T = H | |
| n: | Z = 2-$NO_2$ | T = 4-$OCH_3$ | |
| p: | Z = 2-$NO_2$ | T = 4-$NO_2$ | |
| q: | Z = 4-NH-CO-$CH_2$-CO-$CH_3$ | T = H | |
| r: | Z = H | T = H | R = Phenyl |
| s: | Z = H | T = H | X = N |
| t: | Z = 4-$NO_2$ | T = H | A = Benzo |
| u: | Z = 5-$NO_2$ | T = 2-N($C_2H_5$) | |
| v: | Z = 2-$NO_2$ | T = 4-$COOC_2H_5$ | |
| w: | Z = 2-Cl | T = 4-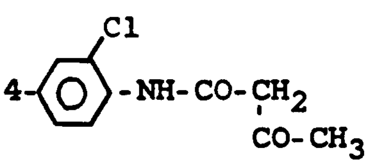 | |
| x: | Z = 2-$NO_2$ | T = 4-$CF_3$ | |
| y: | Z = 5-$CONHC_6H_5$ | T = 2-$OCH_3$ | |
| z: | Z = 4-Cl | T = 3-$SO_2CH_2CH_2OH$ | |
| aa: | Z = 2-$NO_2$ | T = 4-Cl<br>5-O-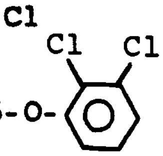 | |

| | | |
|---|---|---|
| ab: | Z = 4-$NO_2$ | T = 2-CN |
| ac: | Z = 4-$NO_2$ | T = 2-Br |
| ad: | Z = 4-$NO_2$ | T = 2-$CF_3$ |
| ae: | Z = 5-$SO_2N(C_2H_5)_2$ | T = 2-$OCH_3$ |
| af: | Z = 3-$OCF_2CF_2H$ | T = H |
| ag: | Z = 4-$NO_2$ | T = 2,5-$Cl_2$ |

Tabelle 2: $^1H$-NMR-Daten der Produkte nach Formel 1

| Produkt (nach Formel (1)) | Enol-Anteil [%] | | Chem. Verschiebung * [ppm] |
|---|---|---|---|
| 1e | 5** | Keto-Form: | 2.35 (s,$CH_3$); 3.63 (s,$CH_2$); 7.25 (t,Ar-$H^4$);*** 7.58 (t,Ar-$H^5$); 7.66 (d,Ar-$H^3$); 8.19 (d,Ar-$H^6$); 9.6 (s,NH) |
| | | Enol-Form: | 2.00 (s,$CH_3$); 5.01 (s,CH); 13.4 (s,OH) |
| 1f | 10 | Keto-Form: | 2.20 (s,$CH_3$); 2.35 (s,$CH_3$); 3.58 (s,$CH_2$; 7.50 (d, Ar-$H^5$); 7.60 (d, Ar-$H^6$); 7.80 (s, Ar-$H^3$); 10.4 (s,NH); |
| | | Enol-Form: | 1.93 (s,$CH_3$); 5.23 (s,CH); 10.2 (s,NH); 13.3 (s,OH) |
| 1h | 8 | Keto-Form: | 2.20 (s,$CH_3$); 3.70 (s,$CH_2$); 3.98 (s,$OCH_3$); 7.79 (d,Ar-$H^3$); 7.87 (dd,Ar-$H^5$); 8.41 (d, Ar-$H^6$); 9.88 (s,NH) |
| | | Enol-Form: | 1.93 (s,$CH_3$); 5.57 (s,CH); 9.6 (s,NH); 13.35 (s,OH) |
| 1k | ca. 2 | Keto-Form: | 2.20 (s,$CH_3$); 3.70 (s,$CH_2$); 7.88 (s,Ar-$H^3$); 8.21 (s,Ar-$H^6$); 9.9 (s,NH) |
| | | Enol-Form: | 1.9 (s,$CH_3$); 5.4 ? (s,CH); 9.67 (s,NH) |

**Fortsetzung Tabelle 2:**

| Produkt (nach Formel (1)) | Enol-Anteil [%] | Chem. Verschiebung * [ppm] |
|---|---|---|
| 1m | 10 | Keto-Form: 2.25 (s,$CH_3$); 3.65 (s,$CH_2$); 7.35 (m,Ar-$H^4$); 7.70 ($\bar{s}$,Ar-$H^{3,5}$, 2H); 7.80 (d,Ar-$H^6$); 10.32 (s,NH) |
| | | Enol-Form: 1.96 (s,$CH_3$); 5.30 (s,CH); 10.7 (s,NH); 13.5 (s,OH) |
| 1n | 10 | Keto-Form: 2.22 (s,$CH_3$); 3.58 (s,$CH_2$); 7.30 (dd,Ar-$H^5$); 7.48 (d,Ar-$H^3$); 7.56 (d,Ar-$H^6$); 10.3 (s,NH) |
| | | Enol-Form: 1.93 (s,$CH_3$); 5.22 (s,CH); 10.0 (s,NH); 13.4 (s,OH) |
| 1p | 12 | Keto-Form: 2.23 (s,$CH_3$); 3.72 (s,$CH_2$); 8.14 (d,Ar-$H^6$); 8.52 ($\bar{d}$,Ar-$H^5$); 8.71 ($\bar{s}$,Ar-$H^3$); 10.95 (s,NH) |
| | | Enol-Form: 1.98 (s,$CH_3$); 5.28 (s,CH); 10.85 (s,NH); 12.9 (s.OH) |
| 1t | 8 | Keto-Form: 2.25 (s,$CH_3$); 3.80 (s,$CH_2$); 7.8 (m,Ar-$H^{6,7}$, 2H); 8.10 (d,Ar-$H^2$); 8.40 (m,Ar-$H^{3,8}$, 2H); 8.50 (d,Ar-$H^5$); 10.45 (s,NH) |
| | | Enol-Form: 2.00 (s.$CH_3$); 5.67 (s,CH); 10.2 (s,NH); 13.6 (s,OH) |

**Fortsetzung Tabelle 2:**

| Produkt (nach Formel (1)) | Enol-Anteil [%] | Chem. Verschiebung * [ppm] |
|---|---|---|
| 1u | 10 | Keto-Form: 2.23 (s,$CH_3$); 2.84 (s,$N(CH_3)_2$); 3.75 (s, $CH_2$); 7.18 (d,Ar-$H^3$); 7.96 (dd,Ar-$H^4$); 8.62 (d,Ar-$H^6$); 9.70 (s,NH)<br>Enol-Form: 1.95 (s,$CH_3$); 5.45 ($\bar{s}$,CH); |
| 1v | 11 | Keto-Form: 1.33 (t,$OCH_2C\underline{H}_3$); 2.23 (s,$CH_3$); 4.32 (q, $OC\underline{H}_2CH_3$); 8.01 (d,Ar-$H^6$); 8.22 (d, Ar-$H^5$); 8.41 (s,Ar-$H^3$); 10.75 (s,NH)<br>Enol-Form: 1.97 (s,$CH_3$); 5.38 (s,CH); 10.6 (s,NH); 13.05 (s,OH) |
| 1x | 10 | Keto-Form: 2.20 (s,$CH_3$); 3.70 (s,$CH_2$); 8.06 (m,Ar-$H^{5,6}$, 2H); 8.30 (s,Ar-$H^3$); 10.78 (s,NH)<br>Enol-Form: 1.98 (s,$CH_3$); 5.27 (s,CH); 10.68 (s,NH); 13.03 (s,OH) |
| 1z | 32**** | Keto-Form: 2.25 (s,$CH_3$); 3.30 (m,$CH_2$); 3.65 (m,$SO_2CH_2$); 3.90 (m, $C\underline{H}_2$-OH); 7.57 (d,Ar-$H^6$); 7.92 (dd,Ar-$H^5$); 8.34 (d,Ar-$H^3$)<br>Enol-Form: 1.90 (s,$CH_3$) |

**Fortsetzung Tabelle 2:**

| Produkt (nach Formel (1)) | Enol-Anteil [%] | | Chem. Verschiebung * [ppm] |
|---|---|---|---|
| 1aa | 10 | Keto-Form: | 2.23 (s,$CH_3$); 3.58 (s,$CH_2$); 7.38 (s,Ar-$H^6$); 7.40 (d,Ar-$H^{6'}$); 7.52 (t,Ar-$H^{5'}$); 7.66 (d, Ar-$H^{4'}$); 8.32 (s,Ar-$H^3$); 10.6 (s,NH) |
| | | Enol-Form: | 1.90 (s,$CH_3$); 5.10 (s,CH); 10.45 ($\bar{s}$,NH); 12.87 (s,OH) |
| 1ab | 9 | Keto-Form: | 2.25 (s,$CH_3$); 3.80 (s,$CH_2$); 8.1 (d,Ar-$H^6$); 8.52 (dd,Ar-$H^5$); 8.75 (d,Ar-$H^3$); 10.75 (s,NH) |
| | | Enol-Form: | 2.00 (s,$CH_3$); 5.35 ($\bar{s}$,CH); 13.2 ($\bar{s}$,OH); |
| 1ac | 9 | Keto-Form: | 2.20 (s,$CH_3$); 3.80 (s,$CH_2$); 8.20 (m,Ar-$H^{5,6}$, 2H); 8.46 ($\bar{s}$,Ar-$H^3$); 10.0 (s,NH) |
| | | Enol-Form: | 1.93 (s,$CH_3$); 5.43 (s,CH); 9.75 (s,NH); 13.2 (s,OH) |
| 1ad | 9 | Keto-Form: | 2.23 (s,$CH_2$); 3.79 (s,$CH_2$); 8.14 (d,Ar-$H^6$); 8.46 (d,Ar-$H^3$); 8.52 (dd,Ar-$H^5$); 10.14 (s,NH) |
| | | Enol-Form: | 1.98 (s,$CH_3$); 5.36 (s,CH) |

**Fortsetzung Tabelle 2:**

| Produkt (nach Formel (1)) | Enol-Anteil [%] | | Chem. Verschiebung * [ppm] |
|---|---|---|---|
| 1ae | 7 | Keto-Form: | 1.05 (t,$NCH_2C\underline{H}_3$); 2.18 (s,$CH_3$); 3.12 (q, $NC\underline{H}_2CH_3$); 3.72 (s,$CH_2$); 3.94 (s,$OCH_3$); 7.22 (d;Ar-$H^3$); 7.50 ($\bar{d}$,Ar-$H^4$); 8.57 ($\bar{s}$,Ar-$H^6$); 9.66 (s,NH); |
| | | Enol-Form: | 1.90 (s,$CH_3$); 5.48 (s,CH); 8.4 (s,Ar-$H^6$) 9.4 (s,NH); 13.45 (s,OH) |
| 1af | 9 | Keto-Form: | 2.20 (s,$CH_3$); 3.58 (s,$CH_2$); 6.77 (tt,$CF_2\underline{H}$) 6.96 ($\bar{d}$,Ar-$H^4$); 7.39 ($\bar{t}$,Ar-$H^5$); 7.41 (m, Ar-$H^6$); 7.71 ($\bar{s}$,Ar-$H^2$); 10.3 (s,NH) |
| | | Enol-Form: | 1.93 (s,$CH_3$); 5.20 (s,CH); 7.68 ($\bar{s}$,Ar-$H^2$) 10.1 (s,NH); 13.6 (s,OH) |
| 1ag | 9 | Keto-Form: | 2.25 (s,$CH_3$); 3.80 (s,$CH_2$); 8.34 (s,Ar-$H^6$); 8.44 (s,Ar-$H^3$); 10.14 (s,NH) |
| | | Enol-Form: | 1.98 (s,$CH_3$); 5.50 (s,CH); 9.88 (s,NH); 13.06 (s,OH) |

**Anmerkungen zu Tabelle 2:**

| | |
|---|---|
| * | Lösungsmittel: DMSO-$d_6$ (falls nicht anders angegeben) |
| ** | 312K in $CDCl_3$ |
| *** | Die Bezifferung der aromatischen Protonen bezieht sich in allen Fällen auf die Acetoacetylaminogruppe |
| **** | in $CD_3OD$ |

**Für die Signalstrukturen sind folgende Abkürzungen eingesetzt:**

**s= Singulett, d= Dublett, t= Triplett, q= Quartett, m= Multiplett, dd= Dublett von Dublett, tt= Triplett von Triplett, $\bar{s}$,$\bar{d}$= jeweils verbreitertes Signal**

**Vergleichsbeispiel 1**

N-(4-Chlor-2-nitrophenyl)acetoacetylamid (Id) in Eisessig ohne Katalysator

86,5 g (0,50 Mol) 4-Chlor-2-nitranilin werden in 500 g Eisessig bei 70 ° C vorgelegt. Innerhalb von etwa 10 Minuten werden 46,0 g (0,55 Mol) Diketen zugetropft. Es erfolgt keine deutlich exotherme Reaktion, und nach insgesamt 1,5 Stunden bei 70 ° C beträgt der Umsatz nach HPLC-Analyse etwa 40 % (Edukt : Produkt wie 60,8 : 39,0/externer Standard).

**Beispiel 30**

N-(4-Chlor-2-nitrophenyl)acetoacetylamid (1d) in Eisessig in Gegenwart von Kaliumfluorid

86,3 g (0,50 Mol) 4-Chlor-2-nitranilin und 2,9 g (0,05 Mol) Kaliumfluorid werden in 500 g Eisessig bei 70 ° C vorgelegt. Innerhalb von 30 Minuten werden 46,2 g (0,55 Mol) Diketen unter Rühren zugetropft. Anschließend wird weitere 2 Stunden bei 70 ° C gerührt und dann, wie in Beispiel 1 beschrieben, aufgearbeitet.
Man erhält 122 g der obengenannten Verbindung (1d) mit einer Reinheit von 96,7 % (HPLC), was einer Ausbeute von 92,0 % der Theorie entspricht.

**Beispiele 31 - 42**

Die in der nachstehenden Tabelle 3 aufgelisteten Umsetzungen wurden, wie in Beispiel 30 beschrieben, durchgeführt. Variiert wurde lediglich der eingesetzte Katalysator. Ausbeute und Reinheit (HPLC) sind in Tabelle 3 angegeben.

**Tabelle 3: Umsetzung von 4-Chlor-2-nitranilin mit Diketen in Eisessig in Gegenwart verschiedener Katalysatoren**

| Beispiel | Katalysator [g] | | Ausbeute (1d) [g] | Reingehalt [%] | Ausbeute (1d) [% d. Th.] |
|---|---|---|---|---|---|
| 31 | 5.1 | Triethylamin | 123,0 | 97,3 | 93,5 |
| 32 | 4,0 | Pyridin | 118,0 | 95,6 | 88,0 |
| 33 | 6,5 | Chinolin | 123,7 | 87,5 | 83,7 |
| 34 | 4,1 | Natriumacetat | 127,6 | 93,9 | 93,4 |
| 35 | 16,2 | Tris(3,6-dioxa-heptyl)amin | 120,2 | 98,5 | 92,3 |
| 36 | 6,1 | N,N-Dimethylanilin | 116,5 | 99,7 | 90,5 |
| 37 | 4,5 | Dimethylamino-ethanol | 110,2 | 99,4 | 85,4 |
| 38 | 7,8 | (Diethylamino)-cyclohexan | 115,3 | 93,9 | 84,4 |
| 39 | 17,7 | Triisoctylamin | 120,3 | 99,1 | 92,9 |
| 40 | 5,8 | N,N,N',N'-Tetra-methylethylendiamin | 123,6 | 99,5 | 95,8 |
| 41 | 6,1 | 4-(Dimethylamino)-pyridin | 124,6 | 98,3 | 95,5 |
| 42 | 5,7 | N-Ethylpiperidin | 124,1 | 98,9 | 95,7 |

**Vergleichsbeispiel 2**

N-(4-Chlor-2-nitrophenyl)acetoacetylamid (1d) in Eisessig in Gegenwart von Natriumchlorid

86,3 g (0,50 Mol) 4-Chlor-2-nitroanilin und 2,9 g (0,05 Mol) Natriumchlorid werden in 500 g Eisessig bei 70 °C vorgelegt. Dann wird weiter, wie in Beispiel 30 beschrieben, verfahren. Man erhält 99,0 g einer Mischung aus der obengenannten Verbindung (1d) und 4-Chloro-2-nitranilin. Der Reingehalt an Verbindung Id beträgt 61,4 % (HPLC), was einer Ausbeute von 47,4 % der Theorie entspricht.

**Patentansprüche**

1. Verfahren zur Herstellung von Acetoacetylarylamiden bzw. -heteroarylamiden desaktivierter Aromaten bzw. Heteroaromaten der allgemeinen Formel (1)

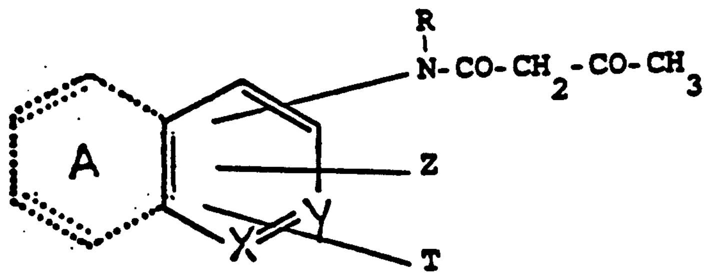

(1)

in welcher X und Y je CH bedeuten oder eines der beiden Ringglieder X und Y ein Stickstoffatom darstellt, A einen anellierten Benzolring bedeutet, der ebenfalls durch T, Z und

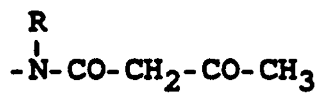

substituiert sein kann, R ein Wasserstoffatom, eine lineare oder verzweigte Alkylgruppe von 1 bis 6 Kohlenstoffatomen, eine Cycloalkylgruppe von 5 oder 6 Kohlenstoffatomen, die durch 1 bis 3 Alkylgruppen von 1 bis 4 Kohlenstoffatomen substituiert sein kann, eine $\beta$-Hydroxyethylgruppe, Benzylgruppe oder eine Phenylgruppe, die durch Fluor-, Chlor-, Brom- und/oder Jodatome und/oder eine Nitrogruppe und/oder lineare oder verzweigte Alkylgruppen und/oder Alkoxygruppen von jeweils 1 bis 4 Kohlenstoffatomen substituiert sein kann, bedeutet, Z ein Fluor-, Chlor-, Brom- oder Jodatom oder eine Gruppe aus der Reihe m-OR′, -OCOR′, $-SO_3R'$, $-SO_2NR'R''$, $-SO_2R'$, -SR′, $-NO_2$, -NH-CO-R′, $-NH-CO-CH_2-CO-CH_3$, $-PO(OR')_2$, $-PO(NR'R'')_2$, -COOR′, -CN, -CONR′R″, -COR′, Halogenalkoxy mit 1 bis 4 Kohlenstoffatomen, Halogenalkyl mit 1 bis 8 Kohlenstoffatomen, wobei Halogen jeweils Fluor, Chlor oder Brom bedeutet, oder eine Phenylgruppe, die durch Fluor-, Chlor-, Brom- und/oder Jodatome und/oder Nitro-, Cyano-, -COR′, -COOR′-Gruppen substituiert sein kann, bedeutet, oder auch ein Wasserstoffatom darstellen kann, wenn X oder Y ein Stickstoffatom oder R in Formel (1) eine gegebenenfalls substituierte Phenylgruppe bedeutet, wobei R′ und R″ Wasserstoffatome, lineare oder verzweigte Alkylgruppen von 1 bis 6 Kohlenstoffatomen, Cycloalkylgruppen von 5 oder 6 Kohlenstoffatomen, die durch 1 bis 3 Alkylgruppen von 1 bis 4 Kohlenstoffatomen substituiert sein können, $\beta$-Hydroxyethylgruppen, Benzylgruppen oder Phenylgruppen, die am aromatischen Kern durch Fluor-, Chlor-, Brom- oder Jodatome und/oder Nitrogruppen und/oder lineare oder verzweigte Alkylgruppen und/oder Alkoxygruppen von jeweils 1 bis 4 Kohlenstoffatomen substituiert sein können, darstellen, T ein Wasserstoffatom, eine oder mehrere lineare oder verzweigte Alkylgruppen oder Alkoxygruppen von 1 bis 6 Kohlenstoffatomen oder eine Cycloalkylgruppe von 5 bis 6 Kohlenstoffatomen, die durch 1 bis 3 Alkylgruppen von 1 bis 4 Kohlenstoffatomen substituiert seinkann, eine Aminogruppe, die durch zwei Alkylgruppen von 1 bis 4 Kohlenstoffatomen substituiert ist, eine $\beta$-Hydroxyethylgruppe oder einen bis zwei, im Falle von Fluor, Chlor und/oder Brom auch mehr als zwei der für Z genannten Substituenten bedeutet, in guten Ausbeuten, und in hoher Reinheit, dadurch gekennzeichnet, daß man 1 Mol eines Amins der allgemeinen Formel (2)

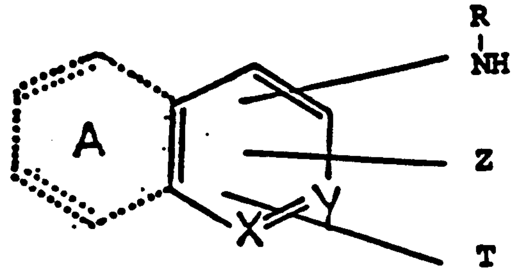

(2)

in welcher A, R, T, X, Y und Z die vorstehend genannten Bedeutungen haben, mit 1 bis 1,2 Mol Diketen bei Temperaturen von 20 bis 100 °C in Eisessig in Gegenwart von 1 bis 20 Molprozent eines basischen Katalysators, bezogen auf das eingesetzte Amin der genannten allgemeinen Formel (2),

umsetzt.

**2.** Verfahren nach Anspruch 1, dadurch gekennzeichnet, daß man als basische Katalysatoren tertiäre Amine verwendet.

**3.** Verfahren nach Anspruch 1, dadurch gekennzeichnet, daß man als basische Katalysatoren Anionen aus der Reihe Fluorid, Acetat, Trifluormethansulfonat, Trifluoracetat, Benzoat, o-, m-, p-Alkyl$C_1$-$C_4$benzoat, o-, m-, p-Alkoxy$C_1$-$C_4$benzoat und o-, m-, p-Di(alkyl$C_1$-$C_4$)aminobenzoat verwendet.

**4.** Verfahren nach Anspruch 2, dadurch gekennzeichnet, daß man als tertiäres Amin (Katalysator) ein Trialkylamin mit Alkylgruppen von 1 bis 9 Kohlenstoffatomen, Cycloalkylgruppen von 5 oder 6 Kohlenstoffatomen, die durch 1 bis 3 Alkylgruppen von 1 bis 4 Kohlenstoffatomen substituiert sein können, Benzylgruppen oder $\beta$-Hydroxyethylgruppen, ein Dialkylarylamin mit Alkylgruppen von 1 bis 6 Kohlenstoffatomen, wobei die Alkylgruppen auch Cycloalkylgruppen von 5 oder 6 Kohlenstoffatomen sein können, die durch 1 bis 3 Alkylgruppen von 1 bis 4 Kohlenstoffatomen substituiert sein können, und wobei die Arylgruppe ein Phenyl-, Alkyl$C_1$-$C_6$phenyl-, Alkoxy$C_1$-$C_4$phenyl- oder Di(alkyl$C_1$-$C_4$)-aminophenyl-Rest sein kann, ferner ein N-Alkyl$C_1$-$C_6$ pyrrolidin, wobei der Alkylrest auch ein Cycloalkylrest von 5 bis 6 Kohlenstoffatomen sein kann, der durch 1 bis 3 Alkylgruppen von 1 bis 4 Kohlenstoffatomen substituiert sein kann, ein N-Alkyl$C_1$-$C_6$piperidin, wobei der Alkylrest auch ein Cycloalkylrest von 5 bis 6 Kohlenstoffatomen sein kann, der durch 1 bis 3 Alkylgruppen von 1 bis 4 Kohlenstoffatomen substituiert sein kann, ferner Pyridin, Monoalkyl$C_1$-$C_4$pyridin, 2,3-, 2,4-, 2,5-, 2,6-, 3,4- oder 3,5-Dialkyl$C_1$-$C_4$pyridin, 2,4,6-Trialkyl$C_1$-$C_4$pyridin, 4-Dimethylamino-pyridin, 4-Diethylamino-pyridin, Chinolin, Isochinolin, N-Alkyl$C_1$-$C_4$morpholin, Triethylendiamin, Tris(3,6-dioxaheptyl)amin oder N,N,N',N'-Tetraalkyl$C_1$-$C_4$alkylen$C_1$-$C_4$diamin verwendet.

**5.** Verfahren nach mindestens einem der Ansprüche 1 bis 4, dadurch gekennzeichnet, daß man 1 Mol eines Amins der in Anspruch 1 genannten allgemeinen Formel (2) mit 1,05 bis 1,1 Mol Diketen bei Temperaturen von 50 bis 80 °C in Gegenwart von 5 bis 10 Molprozent eines basischen Katalysators, bezogen auf das eingesetzte Amin der allgemeinen Formel (2), umsetzt.

**6.** Verfahren nach mindestens einem der Ansprüche 1 bis 5, dadurch gekennzeichnet, daß man die nach Beendigung der Umsetzung vom Produkt der in Anspruch 1 genannten allgemeinen Formel (1) befreite Mutterlauge ganz oder teilweise erneut für das Verfahren einsetzt.

## Claims

**1.** A process for the preparation of an acetoacetylarylamide or -heteroarylamide of a deactivated aromatic or heteroaromatic of the formula (1)

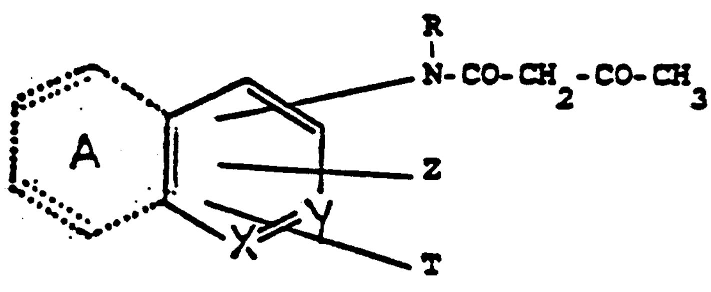

(1)

in which X and Y each denote CH or one of the two ring members X and Y represents a nitrogen atom, A denotes a fused-on benzene ring, which can also be substituted by T, Z and

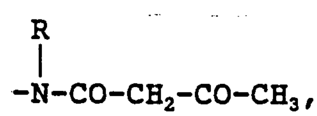

R denotes a hydrogen atom, a linear or branched alkyl group of 1 to 6 carbon atoms, a cycloalkyl

group which has 5 or 6 carbon atoms and can be substituted by 1 to 3 alkyl groups of 1 to 4 carbon atoms, a β-hydroxyethyl group, the benzyl group or a phenyl group which can be substituted by fluorine, chlorine, bromine and/or iodine atoms and/or a nitro group and/or linear or branched alkyl groups and/or alkoxy groups of in each case 1 to 4 carbon atoms, Z denotes a fluorine, chlorine, bromine or iodine atom or a group from the series comprising m-OR', -OCOR', $-SO_3R'$, $-SO_2NR'R''$, $-SO_2R'$,-SR', $-NO_2$, -NH-CO-R', $-NH-CO-CH_2-CO-CH_3$, $-PO(OR')_2$, $-PO(NR'R'')_2$, -COOR', -CN, -CONR'R'', -COR', halogenoalkoxy having 1 to 4 carbon atoms, halogenoalkyl having 1 to 8 carbon atoms, halogen in each case denoting fluorine, chlorine or bromine, or a phenyl group which can be substituted by fluorine, chlorine, bromine and/or iodine atoms and/or nitro, cyano, -COR' or -COOR' groups, or can also represent a hydrogen atom if X or Y denotes a nitrogen atom or R in formula (1) denotes an optionally substituted phenyl group, wherein R' and R'' represent hydrogen atoms, linear or branched alkyl groups of 1 to 6 carbon atoms, cycloalkyl groups which have 5 or 6 carbon atoms and can be substituted by 1 to 3 alkyl groups of 1 to 4 carbon atoms, or β-hydroxyethyl groups, benzyl groups or phenyl groups which can be substituted on the aromatic nucleus by fluorine, chlorine, bromine or iodine atoms and/or nitro groups and/or linear or branched alkyl groups and/or alkoxy groups of in each case 1 to 4 carbon atoms, T denotes a hydrogen atom, one or more linear or branched alkyl groups or alkoxy groups of 1 to 6 carbon atoms or a cycloalkyl group which has 5 to 6 carbon atoms and can be substituted by 1 to 3 alkyl groups of 1 to 4 carbon atoms, or an amino group, which is substituted by two alkyl groups of 1 to 4 carbon atoms, or a β-hydroxyethyl group or one or two, or, in the case of fluorine, chlorine and/or bromine, also more than two, of the substituents mentioned for Z, in a good yield and high purity, which comprises reacting 1 mole of an amine of the general formula (2)

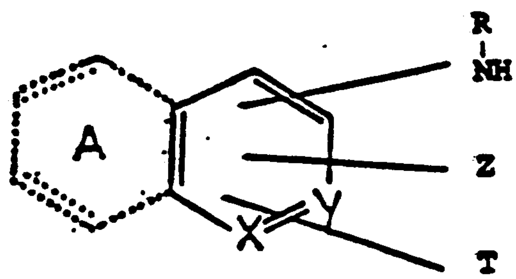

(2)

in which A, R, T, X, Y and Z have the abovementioned meanings, with 1 to 1.2 moles of diketene at temperatures of 20 to 100 ° C in glacial acetic acid in the presence of 1 to 20 mol per cent of a basic catalyst based on the amine of the abovementioned formula (2) employed.

2. The process as claimed in claim 1, wherein a tertiary amine is used as the basic catalyst.

3. The process as claimed in claim 1, wherein an anion from the series comprising fluoride, acetate, trifluoromethanesulfonate, trifluoroacetate, benzoate, o-, m- and p-alkyl$C_1$-$C_4$benzoate, o-, m- and p-alkoxy$C_1$-$C_4$benzoate and o-, m- and p-di(alkyl$C_1$-$C_4$)aminobenzoate is used as the basic catalyst.

4. A process as claimed in claim 2, wherein a trialkylamine with alkyl groups of 1 to 9 carbon atoms, cycloalkyl groups which have 5 or 6 carbon atoms and can be substituted by 1 to 3 alkyl groups of 1 to 4 carbon atoms, benzyl groups or β-hydroxyethyl groups, a dialkyl-arylamine with alkyl groups of 1 to 6 carbon atoms, it also being possible for the alkyl groups to be cycloalkyl groups which have 5 or 6 carbon atoms and can be substituted by 1 to 3 alkyl groups of 1 to 4 carbon atoms, and it being possible for the aryl group to be a phenyl, alkyl$C_1$-$C_6$phenyl, alkoxy$C_1$-$C_4$phenyl or di(alkyl$C_1$-$C_4$)-aminophenyl radical, or an N-alkyl$C_1$-$C_6$pyrrolidine, it also being possible for the alkyl radical to be a cycloalkyl radical of 5 to 6 carbon atoms which can be substituted by 1 to 3 alkyl groups of 1 to 4 carbon atoms, an N-alkyl$C_1$-$C_6$piperidine, it also being possible for the alkyl radical to be a cycloalkyl radical of 5 to 6 carbon atoms which can be substituted by 1 to 3 alkyl groups of 1 to 4 carbon atoms, or pyridine, a monoalkyl$C_1$-$C_4$pyridine, a 2,3-, 2,4-, 2,5-, 2,6-, 3,4- or 3,5-dialkyl pyridine, a 2,4,6-trialkyl$C_1$-$C_4$pyridine, 4-dimethylamino-pyridine, 4-diethylamino-pyridine, quinoline, isoquinoline, an N-alkyl$C_1$-$C_4$morpholine, triethylenediamine, tris(3,6-dioxaheptyl)amine or an N,N,N',N'-tetraalkyl$C_1$-$C_4$-alkylene$C_1$-$C_4$diamine is used as the tertiary amine (catalyst).

5. A process as claimed in at least one of claims 1 to 4, wherein 1 mole of an amine of the formula (2) given in claim 1 is reacted with 1.05 to 1.1 moles of diketene at a temperature of 50 to 80 °C in the presence of 5 to 10 mol per cent of a basic catalyst, based on the amine of the formula (2) employed.

6. The process as claimed in at least one of claims 1 to 5, wherein all or some of the mother liquor freed from the product of the formula (1) given in claim 1 after the reaction has ended is reused for the process.

**Revendications**

1. Procédé pour préparer des acétoacétylarylamides ou des acétoacétyl-hétéroarylamides dérivant de composés aromatiques ou hétéro-aromatiques désactivés, de formule générale (1)

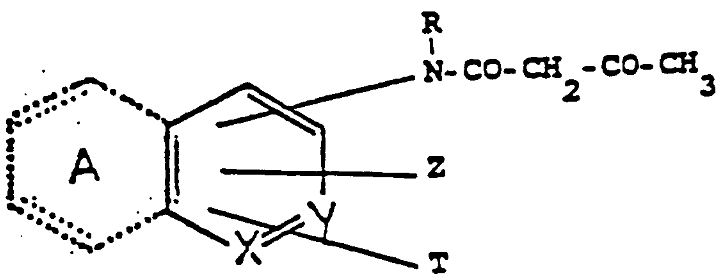

(1)

[dans laquelle X et Y représente chacun CH ou bien l'un des deux chaînons de cycle X et Y représentent un atome d'azote; A représente un noyau benzénique condensé (qui peut éventuellement être substitué par T,Z et

$$-\overset{R}{N}-CO-CH_2-CO-CH_3 \quad ,$$

R représente unn atome d'hydrogène, un groupe alkyle linéaire ou ramifié ayant 1 à 6 atomes de carbone , un groupe cyclo alkyle ayant 1 à 6 atomes de carbone , qui peut être substitué par un à trois groupes alkyles ayant 1 à 4 atomes de carbone, un groupe $\beta$-hydroxyéthyle, un groupe benzyle ou un groupe phényle (qui peut être substitué par des atomes de fluor, de chlore, de brome, et/ou d'iode et/ou par un groupe nitro et/ou par des groupes alkyles et/ou des groupes alcoxy, linéaires ou ramifiés, ayant chacun 1 à 4 atomes de carbone ;

Z représente un atome de fluor, de chlore, de brome ou d'iode, ou un groupe de la série m-OR', -OCOR', - $SO_3R'$, $-SO_2NR'R''$, $-SO_2R'$, -SR', $-NO_2$, -NH-CO-R', $-NH-CO-CH_2-CO-CH_3$, $-PO(OR')_2$, $-PO-(NR'R'')_2$, -COOR', -CN, -CONR", -COR', un groupe halogéno-alcoxy ayant 1 à 4 atomes de carbone , halogéno-alkyle ayant 1 à 8 atomes de carbone, le terme "halogène" désignant à chaque fois un atome de fluor, de chlore ou de brome, ou un groupe phényle (qui peut être substitué par des atomes de fluor, de chlore, de brome et/ou d'iode et/ou par des groupes nitro, cyano, COR', -COOR'), ou bien également Z peut représenter un atome d'hydrogène lorsque X ou y représente un atome d'azote ou quand R représente dans la formule (1) un groupe phényle éventuellement substitué,

R' et R" représentent des atomes d'hydrogène, des groupes alkyles linéaires ou ramifiés ayant 1 à 6 atomes de carbone , des groupes cyclo-alkyles ayant 5 à 6 atomes de carbone , des groupes cyclo-alkyles ayant 5 ou 6 atomes de carbone (qui peuvent être substitués par un à trois groupes alkyles ayant 1 à 4 atomes de carbone), des groupes $\beta$-hydroxyéthyles, des groupes benzyles ou des groupes phényles (qui peuvent être substitués sur le noyau aromatique par des atomes de fluor, de chlore, de brome ou d'iode et/ou par des groupes nitro et/ou par des groupes alkyles et/ou des groupes alcoxy, linéaires ou ramifiés, ayant chacun 1 à 4 atomes de carbone );

T représente un atome d'hydrogène, un ou plusieurs groupes alkyles ou groupes alcoxy linéaires ou ramifiés, ayant 1 à 6 atomes de carbone, ou un groupe cycloalkyle de 5 à 6 atomes de carbone (qui peut être substitué par un à trois groupes alkyles ayant 1 à 4 atomes de carbone ), un groupe amino (qui peut être substitué par deux groupes alkyles ayant 1 à 4 atomes de carbone), un groupe $\beta$-hydroxyéthyle ou un à deux et, dans le cas du fluor, du chlore et/ou du brome également par plus de

deux des substituants cités pour Z), avec de bons rendements ayant une pureté élevée, procédé caractérisé en ce qu'on fait réagir une mole d'une amine de formule générale (2).

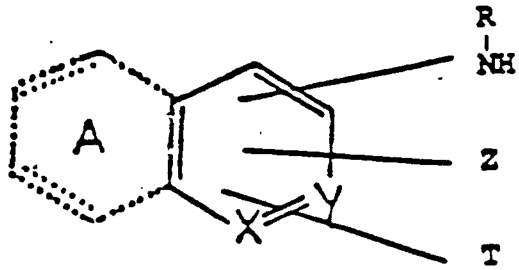

(2)

[dans laquelle A,R,T,X,Y et Z ont les sens précités) avec 1 à 1,2 mole de dicétène à des températures de 20 à 100 °C dans de l'acide acétique cristallisable, en opérant en présence de 1 à 20 moles pour cent d'un catalyseur basique, par rapport à l'amine utilisée, de formule générale (2) citée.

**2.** Procédé selon la revendication 1, caractérisée en ce qu'on utilise comme catalyseurs basiques des amines tertiaires.

**3.** Procédé selon la revendication 1, caractérisé en ce qu'on utilise comme catalyseurs anioniques des anions de la série d'un fluorure, d'un acétate, d'un trifluorométhanesulfonate, d'un trifluoraccétate, d'un benzoate, d'un o-, m-, p- alkyl (en $C_1$ à $C_4$)-benzoate, d'un o-, m-, ou p-alcoxy (en $C_1$ à $C_4$) benzoate et o-, m-, p-di(alkyl en $C_1$ à $C_4$)-amino-benzoate.

**4.** Procédé selon la revendication 2 caractérisé en ce qu'on utilise comme amine tertiaire (catalyseur) une trialkylamine comportant des groupes alkyles ayant 1 à 9 atomes de carbone , des groupes cycloalkyles ayant 5 ou 6 atomes de carbone (qui peuvent être substitués par un à trois groupes alkyles ayant 1 à 4 atomes de carbone ), des groupes benzyles ou des groupes $\beta$-hydroxyéthyles, une dialkylarylamine comportant des groupes alkyles ayant 1 à 6 atomes de carbone , les groupes alkyles pouvant également être des groupes cycloalkyles comportant 5 ou 6 atomes de carbone et qui peuvent être substitués par un à trois groupes alkyles ayant 1 à 4 atomes de carbone , et le groupe aryle pouvant être un groupe phényle, alkyl (en $C_1$ à $C_6$) phényle, alcoxy (en $C_1$ à $C_4$) phényle ou di(alkyl en $C_1$ à $C_4$)-aminophényle, ainsi qu'une N-alkyl(en $C_1$ à $C_6$)pyrrolidine, le reste alkyle pouvant également être un reste cycloalkyle comportant 5 à 6 atomes de carbone et qui peut être substitué par un à trois groupes alkyles ayant 1 à 4 atomes de carbone , une N-alkyl(en $C_1$ à $C_6$)pipéridine, le reste alkyle pouvant également être un reste cycloalkyle ayant 5 à 6 atomes de carbone et qui peut être substitué par un à trois groupes alkyles ayant 1 à 4 atomes de carbone, ainsi que la pyridine, une monoalkyl(en $C_1$ à $C_4$) pyridine, une 2,3-, une 2,4, une 2,5-, une 2,6-, une 3,4- ou une 3,5-dialkyl (en $C_1$ à $C_4$)-pyridine, une 2,4,6-trialkyl(en $C_1$ à $C_4$)pyridine, la 4-diméthylamino-pyridino, la 4-diéthylamino-pyridine, la quinoléine, l'isoquinoléine, une N-alkyl (en $C_1$ à $C_4$)morpholine, la triéthylenediamine, la tris(3,6-dioxaheptyl)amine ou une N,N,N',N'-tétralkyl(en $C_1$ à $C_4$) alkylène (en $C_1$ à $C_4$)diamine.

**5.** Procédé selon l'une au moins des revendications 1 à 4, caractérisé en ce qu'on fait réagir une mole d'une amine, de formule générale (2) citée à la revendication 1, avec 1,05 à 1,1 mole de dicétène à des températures de 50 à 80 °C en présence de 5 à 10 moles pour cent d'un catalyseur basique par rapport à l'amine de formule générale (2) que l'on utilise.

**6.** Procédé selon l'une au moins des revendications 1 à 5, caractérisé en ce qu'on utilise à nouveau pour le procédé, entièrement ou partiellement, la liqueur mère, débarrassée, après achèvement de la réaction, du produit de formule générale (1) cité à la revendication 1.